(19) Europäisches Patentamt / European Patent Office / Office européen des brevets

(11) **EP 4 655 582 B1**

(12) **EUROPEAN PATENT SPECIFICATION**

(45) Date of publication and mention
of the grant of the patent:
**29.04.2026 Bulletin 2026/18**

(21) Application number: **24705737.5**

(22) Date of filing: **23.01.2024**

(51) International Patent Classification (IPC):
*G01N 27/02* (2006.01)    *G01N 27/48* (2006.01)
*G01N 33/49* (2006.01)    *G01N 15/06* (2024.01)
*G01N 15/00* (2024.01)

(52) Cooperative Patent Classification (CPC):
**G01N 15/0656; G01N 33/49; G01N 33/492;**
G01N 15/01

(86) International application number:
**PCT/IB2024/050613**

(87) International publication number:
**WO 2024/157161 (02.08.2024 Gazette 2024/31)**

(54) **METHOD FOR DETECTING AND/OR QUANTIFYING, IN A SAMPLE OF BLOOD OR DERIVATE BIOLOGICAL LIQUID, A PANEL OF HAEMATOLOGICAL PARAMETERS**

VERFAHREN ZUR DETEKTION UND/ODER QUANTIFIZIERUNG EINER PLATTE VON HÄMATOLOGISCHEN PARAMETERN IN EINER BLUT- ODER DERIVAT-BIOFLÜSSIGKEIT

PROCÉDÉ DE DÉTECTION ET/OU DE QUANTIFICATION D'UN PANEL DE PARAMÈTRES HÉMATOLOGIQUES DANS UN ÉCHANTILLON SANGUIN OU UN LIQUIDE BIOLOGIQUE DÉRIVÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **24.01.2023 IT 202300001014**

(43) Date of publication of application:
**03.12.2025 Bulletin 2025/49**

(73) Proprietor: **FIDELIO MEDICAL S.R.L.**
**10121 Torino (IT)**

(72) Inventors:
• **CHIARA, Francesco**
**10121 Torino (IT)**
• **ARDUINI, Fabiana**
**10121 Torino (IT)**
• **FIORE, Luca**
**10121 Torino (IT)**
• **ROGGERO, Simona**
**10121 Torino (IT)**

(74) Representative: **Studio Torta S.p.A.**
**Via Viotti, 9**
**10121 Torino (IT)**

(56) References cited:
WO-A1-2019/124917    WO-A1-2019/124917
WO-A1-94/29731       WO-A1-94/29731
US-A1- 2002 048 532  US-A1- 2002 048 532
US-A1- 2013 062 221  US-A1- 2013 062 221
US-A1- 2017 045 469  US-A1- 2017 045 469
US-A1- 2019 154 620  US-A1- 2019 154 620
US-A1- 2020 278 314  US-A1- 2020 278 314

**Description**

<u>Cross-Reference to Related Applications</u>

This Patent Application claims priority from Italian Patent Application No. 102023000001014 filed on January 24, 2023.

<u>Technical Field of the Invention</u>

The present invention relates to a method for detecting and/or quantifying, in a sample of blood or derivate biological liquid, a panel of haematological parameters comprising at least one parameter selected from the group consisting of the number of corpuscular elements in the blood or derivate biological liquid (RC), mean corpuscular volume of said corpuscular elements (MCV), percentage of the volume of sample occupied by the corpuscular elements (HCT) and amount of at least one substance comprising a protoporphyrin metal complex (Hb).

<u>Background</u>

Haemoglobin (Hb) is a tetrameric metalloprotein found in red blood cells (RBCS), it contains four iron atoms essential for the transport of oxygen from the lungs to the body tissues. The same molecule also takes charge of conveying a part of the carbon dioxide from the tissues that produced it to the lungs. Conventionally, the measurement of the concentration of Hb in the blood is a specific and sensitive indicator for the diagnosis of anaemia and other diseases correlated to disorders of the level of Hb in the blood. Normal haemoglobin values are strictly dependent on the age and sex of the subject. The conventional method used for the measurement of the haemoglobin is the Drabkin test, developed in 1930. The detection is based on the formation of the cyanohemoglobin through interaction between the cyanide ions and the oxidized haemoglobin. The dosage takes place by spectrophotometric measurement of absorbance, at a wavelength of 540 nm, corresponding to the characteristic absorbance peak of the cyanohemoglobin. Over the years, other methods for the determination of haemoglobin have been developed, mainly colorimetric and chromatographic methods such as fluorimetry, colorimetry, HPLC and chemiluminescence. Although these techniques allow the quantification of low concentrations of haemoglobin, they are subject to some disadvantages:

- bulky instrumentation (does not allow analysis at the point-of-care or in poorly equipped medical clinics);
- expensive instrumentation;
- high analysis time;
- use of highly qualified personnel;
- complex analysis processes;
- use of expensive materials and reagents.

All these disadvantages have stimulated research to develop new techniques to overcome or reduce these problems. Electrochemical methods are a particularly promising alternative for the analysis of haemoglobin in biological liquids. Depending on the type of modification made on the electrode surface, these methods can be subdivided into four main classes:

1) immobilization of haemoglobin on the electrode surface;
2) use of organic and inorganic catalysts;
3) use of molecular imprinted polymer (MIP) membranes;
4) immunological methods.

The well known prior art document is the study conducted by Brett C.M. *et al.* 1999, which consists in the development of an electrochemical sensor suitable for monitoring haemoglobin in whole blood by using methylene blue as an electrochemical mediator. In this study, a glassy carbon electrode consisting of a classic three-electrode system is exploited. In detail, the electrochemical cell is composed of a graphite working electrode, a calomel reference electrode and a platinum counterelectrode. The cell is filled with a 0.1 M phosphate or acetate buffer solution which acts as a supporting electrolyte.

The electrochemical mediator (methylene blue) is electrochemically deposited on the surface of the working electrode by immersing the sensor into a beaker containing a solution composed of 0.2 mM of methylene blue, 0.1 M of phosphate buffer (pH=8.2) and 0.1 M of $Na_2SO_4$. The electrochemical deposition is carried out by applying a potential scan in a range between -0.6 and 1 V, performing 20 scans in cyclic voltammetry. As soon as the electrodeposition is carried out, the sensor is left immersed in an electrolytic solution for a few hours or overnight, this allows for the elimination of the unpolymerized monomer and therefore a more stable electrochemical measurement. The haemoglobin is determined by batch injection analysis (BIA) technique, which allows the measurement of the solution contained in the beaker wherein the sensor is immersed, after subsequent additions of whole blood (about 50 μL). The signal transduction takes place by chronoamperometry; a current signal proportional to the concentration of haemoglobin present in the solution is obtained by applying a potential of +550 V. In order to reduce the memory effect, caused by a possible retention or absorption of metalloprotein within the film deposited on the electrode, with the BIA technique 20 sec are awaited before carrying out the subsequent addition, so that the residual current value stabilizes. In this study, 8 whole blood samples were analysed, which were taken from 8 voluntary donors. This matrix, treated immediately with EDTA to prevent coagulation, was diluted 1:5 with phosphate buffer prior to performing the electrochemical measurement. Six of the samples analysed gave results comparable with the dosage of the haemoglobin performed by means of the reference method.

Document US2013062221A1 discloses a method for detecting hemoglobin combining measurements of EIS and chronoamperometry in the presence of a mediator.

All the methods described above have disadvantages, among which a common problem is the impossibility of carrying out haemoglobin detection, on whole blood without carrying out other treatments. Several of the aforesaid methods then require expensive and large-sized instrumentation, equipped laboratories, qualified personnel, a considerable execution time, limited specificity and selectivity.

In addition to the quantification of the haemoglobin, the need is felt to also measure a whole other series of haematological parameters without resorting to the analysis laboratory and by means of minimum amounts of biological liquid. Some of these parameters are cell elements of the blood such as red blood cells, reticulocytes, erythroblasts, in particular their count, mean corpuscular volume and relative volume, and substances containing (proto)porphyrin metal complexes such as haemoglobin, myoglobin, zinc protoporphyrin.

There is therefore a need to develop a method that allows to detect and quantify the number of corpuscular elements in the blood or in a derivate biological liquid, the mean corpuscular volume of said corpuscular elements, the percentage of the volume of sample occupied by corpuscular elements and/or the amount of at least one substance comprising a protoporphyrin metal complex, which can be applied both outside the laboratory, and in the laboratory in emergency situations or in the case of small and poorly equipped facilities. In particular, there is a strong demand to provide a method that is less expensive, faster and simpler, specific and selective and adaptable to laboratories and non-specialised environments from a diagnostic point of view.

## Summary of the Invention

It is therefore an object of the present invention to provide a method for detecting and/or quantifying, in a sample of blood or derivate biological liquid, a panel of haematological parameters comprising at least one parameter selected from the group consisting of the number of corpuscular elements in the blood or derivate biological liquid (RC), mean corpuscular volume of said corpuscular elements (MCV), percentage of the volume of sample occupied by the corpuscular elements (HCT) and amount of at least one substance comprising a protoporphyrin metal complex (Hb), which allows to solve the aforesaid problems in a simple and efficient manner.

This object is achieved by the present invention as it relates to a method as defined in claims 1 and 2.

## Brief description of the figures

Figure 1 represents a schematic view of an example of electroanalytical sensor not according to an embodiment of the present invention.

Figure 2 represents a graph of the cyclic voltammetry (CV) obtained by measuring 10 μl of solution containing 2mM of methylene blue, 0.1 M phosphate buffer solution (pH 7.4) and 0.1 M $Na_2SO_4$.

Figure 3 represents a graph wherein the impedance (Z) is on the abscissa axis thereof and the surface factor (St) multiplied by the corresponding current (i) is on ordinate axis thereof.

Figure 4 represents a graph wherein the concentration of haemoglobin ([Hb]) is on the abscissa axis thereof and the surface factor (St) multiplied by the impedance (Z) decreased by the current value (i) is on the ordinate axis thereof.

Figure 5 represents a graph wherein the haematocrit value (HCT) is on the abscissa axis thereof and the surface factor (St) multiplied by the impedance (Z) decreased by the current value (i) is on the ordinate axis thereof.

Figure 6 represents a graph wherein the count of the red blood cells (RC) is on the abscissa axis thereof and the surface factor (St) multiplied by the impedance (Z) decreased by the current value (i) is on the ordinate axis thereof.

Figure 7 represents a graph wherein the concentration of haemoglobin [Hb] normalized to the corresponding impedance (Z) as a function of the current (i) is shown.

Figure 8 represents a graph showing how the impedance measurement (Z) corrected by the factor of electroactive surface (St), correlates with a fair significance with the concentration of haemoglobin expressed in mol/L (M). The scale of the abscissa axis is logarithmic.

Figure 9 represents the comparison between the new measurement model of the concentration of blood haemoglobin and the concentration of haemoglobin determined with the reference method.

Figure 10 represents a graph wherein there are shown as a natural logarithm the resistance measurements ($\Delta$V/I) normalized to the count of red blood cells contained in the volume of capillary blood deposited on the surface of the electrochemical cell (60 $\mu$l), as a function of the impedance measurements.

Detailed description of the invention

The method according to the invention for detecting and/or quantifying, in a sample of blood or derivate biological liquid, a panel of haematological parameters comprising at least one parameter selected from the group consisting of the number of corpuscular elements in the blood or derivate biological liquid (RC), mean corpuscular volume of said corpuscular elements (MCV), percentage of the volume of sample occupied by the corpuscular elements (HCT) and amount of at least one substance comprising a protoporphyrin metal complex (Hb), comprises the following steps.

In a first step the sample of blood or derivate biological liquid is applied to an electroanalytical sensor comprising at least one first working electrode, a reference electrode and a counterelectrode.

In a second step, a current signal is detected, by chronoamperometry, which is a function of the amount of substance comprising the protoporphyrin metal complex in the blood or derivate biological liquid by means of the electroanalytical sensor, wherein an electrochemical mediator, which remains insoluble during the execution of the method, has been electrodeposited on said at least one working electrode. In a third step, an impedance signal is detected, by electrochemical impedance spectroscopy, which is a function of at least the number of corpuscular elements in the blood or derivate biological liquid. More precisely, the electronic circuit that allows the execution of the electrochemical impedance spectroscopy allows the measurement of a resistive quantity called Rs, which corresponds to the transport of charge in solution and is closely correlated to impedance. For this reason and for simplicity's sake, this parameter will be referred to herein with the more generic term of "impedance".

In a fourth step, the at least one parameter of the panel is quantified by means of at least one specific mathematical formula wherein the at least one parameter is a function of the current signal and of the impedance signal.

The sample is preferably venous or capillary blood. By the definition derivate biological liquid it is intended to comprise the different fractions of the blood, as well as solutions comprising blood and anticoagulant substances.

By corpuscular elements it is intended to mean cell elements selected from the group consisting of erythrocytes, reticulocytes and erythroblasts.

The protoporphyrin metal complex is preferably an iron porphyrin metal complex, more preferably haemoglobin or myoglobin, or a zinc porphyrin metal complex, more preferably zinc protoporphyrin.

The percentage of the volume of sample occupied by corpuscular elements (HCT) is preferably the haematocrit. The number of corpuscular elements in the blood or derivate biological liquid (RC) is preferably the number of erythrocytes. The amount of at least one substance comprising a protoporphyrin metal complex (Hb) is preferably the concentration of haemoglobin.

The sample of blood or derivate biological liquid preferably has a volume smaller or equivalent to 50 $\mu$l, more preferably smaller than 15 $\mu$l.

Preferably, the panel of parameters comprises two or more said parameters.

The method comprises the step of detecting by chronoamperometry and the step of detecting by electrochemical impedance spectroscopy. The steps of detecting by chronoamperometry and electrochemical impedance spectroscopy may be successive or simultaneous, preferably simultaneous.

Preferably, the step of detecting by chronoamperometry is carried out at a potential from -1V to +1V, more preferably at a potential of about 0.55 V.

By electrochemical mediator is meant a substance subject to oxidoreduction equilibria such that they spontaneously reduce or oxidize the analyte, generating a current at the interface with the working electrode at a given range of potential within the context of an electroanalytical detection system.

The electrochemical mediator is defined insoluble in that, at the time the electrodeposition is performed and then the mediator is adsorbed on the electrode surface, it remains insoluble in the matrix (e.g., the blood) during the execution of the measurement. On the other hand, when the drop casting is performed (like it happens in the prior art) the potassium ferrocyanide becomes soluble when the measurement of the haemoglobin is performed.

Preferably, the insoluble electrochemical mediator is electrodeposited on the at least one working electrode. This allows to avoid using ligands to trap the mediator to the working electrode.

Preferably, the insoluble electrochemical mediator is polymerizable.

Preferably, the insoluble electrochemical mediator is selected from the group consisting of methylene blue, methyl viologen, o-cresol indophenol, 2-6 dibromophenol indophenol, toluidine blue, haematoxylin/eosin, and periodic acid with Schiff stain. More preferably, the insoluble electrochemical mediator is methylene blue.

In one method according to the invention, the parameter amount of at least one substance comprising a protoporphyrin metal complex (Hb) is measured and the specific mathematical formula is:

$$[Hb] = Z \cdot (\alpha_4 \cdot i + \beta_4)$$

wherein [Hb] is the concentration of the substance comprising the protoporphyrin metal complex; and wherein $\alpha_4$ and $\beta_4$ are respectively the slope and the intercept of the fitting line to the least squares obtained placing the ratio between the concentration of the substance comprising the protoporphyrin metal complex and the impedance on the ordinate axis, and the current intensity on the abscissa axis.

In the specific mathematical formula of the other method the at least one parameter is a function of the total surface of all the corpuscular elements (St).

The total surface of the corpuscular elements (St) represents the ideal total surface, having assumed that the total morphology of the red blood cells is spherical.

Assuming that in a broad sense the cell and corpuscular elements such as erythroblasts, reticulocytes and red blood cells have a spherical rather than toroidal morphology, we can proceed by writing

$$MCV \sim 4/3 \cdot \pi r^3$$

where MCV is the mean corpuscular volume expressed in fL (femtolitres), r the radius of the cell/corpuscle whose morphology is assimilated to a sphere.

Therefore, knowing that r = d/2, where r is the spherical radius and d the relative diameter, substituting in the above equation and solving with respect to d we obtain

$$d = \sqrt[3]{(6/\pi \cdot MCV)}$$

It is in fact convenient to consider the diameter rather than the radius, since the red blood cells stack on top of each other and on the electrode.

Considering that the spherical surface is given by $S = 4\pi r^2$, it can be easily verified that the surface of the cell/i-th corpuscle present on the surface of the sensor has the i-th spherical surface given by

$$S_i = \sqrt[3]{(36\pi \cdot (MCV)^2)}$$

Considering also that the total surface of the cells/corpuscles interacting on the electroactive surface depends on the volume of the drop of blood/derivate biological liquid deposited on the sensor, on the haematocrit (percentage volume of red blood cells in the blood), as well as on the number of erythrocytes as such, it can be postulated that St (ideal total surface of interaction) is given by

$$S_t \propto RC \cdot HCT \cdot V$$

where RC is the count of red blood cells, HCT the haematocrit and V the volume of blood/derivate biological liquid deposited on the sensor.

Reminding that the units of measurement commonly used in the hemochromocytometric examination are not those of the SI, it is necessary to use a conversion factor that is defined as k and that is equal to $4.83598 \cdot 10^{-6}$, expressing St in $m^2$.

Therefore, it is possible to obtain

$$S_t = RC \cdot HCT \cdot V \cdot \sqrt[3]{(36\pi \, (MCV)^2)}$$

RC is the number of said corpuscular elements $[n. \cdot 10^{12}/l]$
HCT is the relative corpuscular volume of said corpuscular elements [%V/V]
V is the volume of the sample of blood or derivate biological liquid applied to the sensor
MCV is the mean corpuscular volume [fl].
St is a function of the impedance signal and of the current signal by means of the equation

$$S_t = (\alpha_3 \cdot Z + \beta_3)/i$$

wherein $\alpha_3$ and $\beta_3$ are respectively the slope and the intercept of a calibration curve obtained experimentally and traced on a graph wherein Z ($\Omega$) is on the abscissa axis and St $\cdot$ i ($m^2 \cdot \mu A$) is on the ordinate axis.

In a preferred embodiment of the method according to the invention, the parameter amount of at least one substance comprising a protoporphyrin metal complex (Hb) is measured and the specific mathematical formula is:

$$[Hb] = (S_t \bullet Z - \beta_0 - i)/\alpha_0$$

wherein [Hb] is the concentration of the substance comprising the protoporphyrin metal complex; and
$\alpha_0$ and $\beta_0$ are respectively the slope and the intercept of a calibration curve obtained experimentally and traced on a graph wherein [Hb] (g/dL) is on the abscissa axis and (St $\cdot$ Z-i) ($m^2$ * Ohm - $\mu A$) is on the ordinate axis.

In another preferred embodiment of the method according to the invention, the percentage parameter of volume occupied by corpuscular elements/volume of the sample is measured and the specific mathematical formula is:

$$HCT = (S_t \bullet Z - \beta_1 - i)/\alpha_1$$

wherein HCT is the relative corpuscular volume of said corpuscular elements; and
$\alpha_1$ and $\beta_1$ are respectively the slope and the intercept of a calibration curve obtained experimentally and traced on a graph wherein HCT (%v/v) is on the abscissa axis and (St $\cdot$ Z-i) ($m^2$ * Ohm - $\mu A$) is on the ordinate axis.

In another preferred embodiment of the method according to the invention, the parameter number of corpuscular elements in the blood or derivate biological liquid is measured and the specific mathematical formula is:

$$RC = (S_t \bullet Z - \beta_2 - i)/\alpha_2$$

wherein RC is the number of said corpuscular elements; and
$\alpha_2$ and $\beta_2$ are respectively the slope and the intercept of a calibration curve obtained experimentally and traced on a graph wherein RC (n. corpuscular elements $\cdot$ $10^{12}$/l) is on the abscissa axis and ($S_t \cdot$ Z-i) ($m^2$ * Ohm - $\mu A$) is on the ordinate axis.

In another preferred embodiment of the method according to the invention, the parameter mean corpuscular volume of the corpuscular elements in the blood or derivate biological liquid is measured and the specific mathematical formula is:

$$MCV = 1/(6 \cdot \sqrt{\Pi}) \bullet (S_t/(\cdot RC \cdot HCT \cdot V))^{(3/2)}$$

wherein MCV is the mean corpuscular volume.

In a preferred embodiment of the method according to the invention, all parameters of the panel are measured.

The method according to the invention has numerous applications of use: drug monitoring, screening, diagnosis and monitoring of haematological pathologies, monitoring in sports subjects.

Examples

Example 1 - Configuration of the sensor with screen-printed electrodes

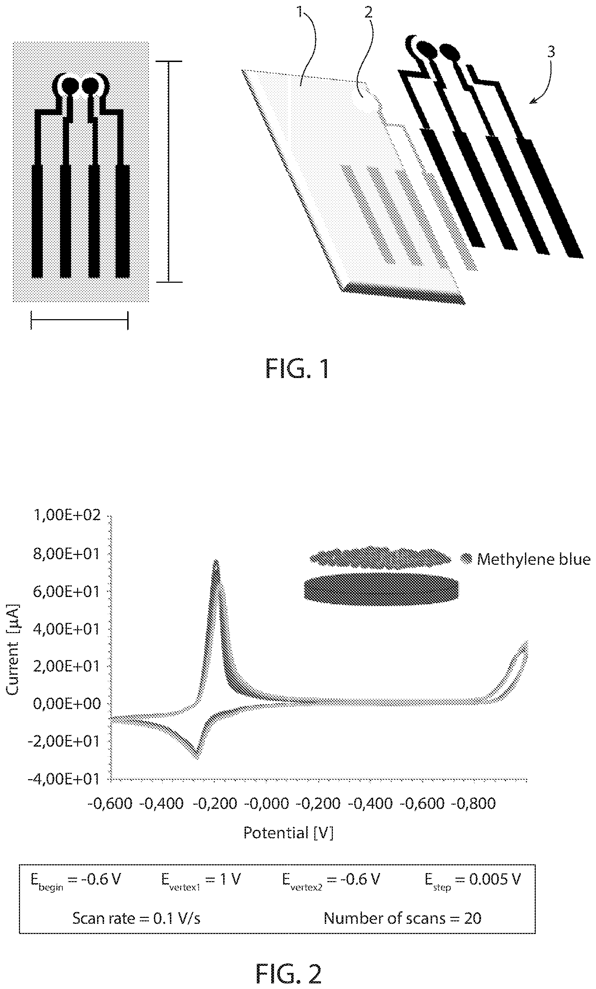

Screen-printed electrodes (SPEs) were produced using transparent and flexible polyester (or paper) as a support and 245 DEK (Weymouth, UK) as a screen-printing device. A four-electrode cell was manufactured using graphite-based ink for the two working electrodes and the counterelectrode, and an ink based on silver or silver/silver chloride (Ag/AgCl) for the pseudo reference electrode. This new configuration can simultaneously detect the haemoglobin and the correlated blood parameters, such as haematocrit, MCV, count of the red blood cells, and iron. The sensor is shown in Figure 1 wherein reference numeral 1 denotes the polyester support, reference numeral 2 denotes the reference electrode in Ag/AgCl ink, and reference numeral 3 denotes the first working electrode, the second working electrode, and the counterelectrode, all in graphite ink.

Example 2 - Modification of the screen-printed electrode

The first working electrode was modified with methylene blue (MB) by electrochemical deposition. MB is an electrochemical mediator capable of selectively oxidizing Fe(II) coordinated with haemoglobin heme. In detail, the electrodeposition was performed in cyclic voltammetry (CV) using the parameters specified in Figure 2. CV cycles were performed by measuring 10 $\mu$l of solution containing 2 mM methylene blue, 0.1 M phosphate buffer (pH 7.4) and 0.1 M $Na_2SO_4$. The results are shown in Figure 2.

Example 3 - Electrochemical measurement

In the blood matrix, haemoglobin is bound to the plasma membrane of the red blood cells through a protein cluster that involves several specific membrane proteins. Haemoglobin is directed by polarization phenomena and at the same time by the diffusion of the red blood cells within the volume of blood deposited on the surface of the electrochemical cell. For these reasons, the haemoglobin was determined by applying two different techniques: chronoamperometry (CA) and electrochemical impedance spectroscopy (EIS). Through CA it is possible to obtain the oxidation of iron (II) by interacting with methylene blue, which in turn is reduced to semi-quinone form. The current value obtained from the CA measurement is then associated with the EIS response, which is determined by the volume and by count of red blood cells.
In detail, CA and EIS were carried out by applying the following parameters:

| CA | EIS |
|---|---|
| $E_{dc}$ = 0.55 V | Scan type = fixed |
| $T_{internal}$ = 0.1 s | $E_{dc}$ = 0 V |
| $T_{run}$ = 60 s | $E_{ac}$ = 0.01 V |
| | Frequency type = scan |
| | N frequency = 41 = 10 / dec |
| | Maximum frequency = 10000.0 Hz |
| | Minimum frequency = 1.0 Hz |

The measurements were performed by depositing a volume of blood between 4 and 10 $\mu$l in order to wet the electrochemical cell.

Example 4 - Electrochemical detection of the haemoglobin

Haemoglobin is among the substances that contain protoporphyrin metal complexes, in the specific case Fe2-protoporphyrin (IX).
In the specific case of the haemoglobin, the invention provides that the first working electrode is modified by electrodeposition with at least one redox mediator of Fe(II)-protoporphyrin(IX) metal complexes and with chemicalphysical characteristics that allow the electrodeposition: preferably methylene blue, methyl viologen, o-cresol indophenol, 2-6 dibromophenol-indophenol.
Zinc-protoporphyrin is among the substances that contain protoporphyrin metal complexes, in this specific case Zn2-protoporphyrin(IX).
In the specific case of the haemoglobin, our invention provides that the first working electrode is modified by electrodeposition with at least one redox mediator of Zn(II) - protoporphyrin(IX) metal complexes, preferably diphenylbenzidine.
Haemoglobin anchored to the plasma membrane of the blood cells, presents in its optimal conformation for oxygen binding, an oxidation state (+2) of the iron coordinated in the four protoporphyrin structures in a 1:4 stoichiometric ratio in each haemoglobin molecule itself. Iron (+2) allows its own detection by oxidation at a potential of +0.55V using an electrochemical cell on solid state, where the working electrode is suitably functionalised with an electrochemical mediator that allows the optimisation of the kinetics of the oxidation-reduction currents at the interface with the electrode itself.
Heme iron, in its optimal state, is reduced (II). Apohaemoglobin is composed of four subunits containing a protoporphyrin ring bound to iron (II) in a 1:4 stoichiometric ratio. The sensor is based on the detection of iron (II) through oxidation with the mediator methylene blue as follows:

$$MB+nFe^{2+} \leftrightarrow (S+L) +nFe^{3+}MB+nFe^{2+} \leftrightarrow S+L+nFe^{3+}$$

where MB is methylene blue which is reduced to semiquinone form (S) and in leuco-methylene blue (L). One of the two species prevails as a function of the pH shift.

In other words, the aforesaid reaction represents the redox reaction between heme iron (II) and methylene blue (MB) which is reduced into two molecular forms: semiquinone (S) and leuco quinone (L). The working electrode (modified with methylene blue) is able to detect iron (II) at an applied potential of + 0.55 V.

Now, the iron (II) coordinated to haemoglobin heme is certainly protected from oxidation by antioxidant mechanisms (glutathione etc.) and by molecular mechanisms that involve the conformation of the protein. Therefore, an oxidation process at the electrode of an electrochemical cell cannot take place in a "simple" form but it must be "guided" with the use of a mediator such as methylene blue or analogues thereof.

A further characteristic, already mentioned, is that haemoglobin is anchored to the plasma membrane of the blood cells through a sort of protein cluster that involves several specific membrane proteins such as some spectrins, ankyrin, etc.

These two phenomena pose the experimental problem of being able to detect the haemoglobin within a context of a complex system such as blood, where our analyte is "protected" either by the conformation itself of the protein, or by its bond to a structure that makes it more dynamic in space and in time as befits a cell (the red blood cell).

This type of "interferences" do not take long to emerge when one is about to detect haemoglobin with an electrochemical system consisting of a three-electrode cell printed on a polyester surface. In this condition the working electrode of the cell is functionalized with methylene blue by electrodeposition. The correlation between currents determined by applying a potential of 0.55 V for 60s and performing a measurement on blood samples whose concentration of haemoglobin has been previously determined with the clinical reference method 1, is not possible: the data appear absolutely random.

In this context, the already mentioned phenomena should be recalled and above all it is necessary to investigate the mechanisms of interaction between the matrix and the mediator. Certainly, haemoglobin is conveyed by polarization phenomena and at the same time by diffusion of the red blood cells within the volume of blood deposited on the surface of the electrochemical cell, and in particular on the working electrode. Thus, if the carrier are the cells, the interaction of the protein with the mediator will depend on steric factors of the cells themselves and on the geometry of distribution of the same cells on the electrode surface. As is generally known, a surface factor is inevitably linked to the volume of the body being considered, and at such a point if the bodies are many and all the same, the sum of the interactions will depend on the number of the bodies present.

Therefore, considering the hemochromocytometric examination, it is possible to macroscopically identify the series of parameters that are needed to be able to estimate the interactions to which mention was made: MCV (mean corpuscular volume), HCT (haematocrit), RBC (number of erythrocytes).

Thus, what has been defined so far as the interaction between the cells transporting the analyte and the functionalised surface of the working electrode, can be better defined as the estimate of the electroactive surface (of the matrix) at the working electrode.

Following several observations (see explanation above) it can be stated that the total electroactive surface of the red blood cells can be estimated analytically as

$$S_t = RC \cdot HCT \cdot V \cdot \sqrt[3]{(36\pi \ (MCV)^2)}$$

where

RC is the number of said corpuscular elements [$n. \cdot 10^{12}$ /l]
HCT is the relative corpuscular volume of said corpuscular elements [%V/V]
V is the volume of the sample of blood or derivate biological liquid applied to the sensor
MCV is the mean corpuscular volume [fl].

In order to be able to verify whether the hypotheses set out so far and supported by this first evidence were correct, it was necessary to investigate the matrix with an impedance measurement technique, thus measuring the impedance through a second working electrode.

Having verified in this regard that the impedance values obtained and subsequently normalized by the count of red blood cells contained in the volume deposited on the electrode surface, were correlable to the MCV, we proceeded with studying the correlation between concentration of haemoglobin and the other parameters contemplated in the hemochromocytometric examination.

The measurements of the count of red blood cells (RC), haematocrit (HCT), haemoglobin, and mean corpuscular volume (MCV) can be derived from the measurement of the surface factor (St) as indicated below.

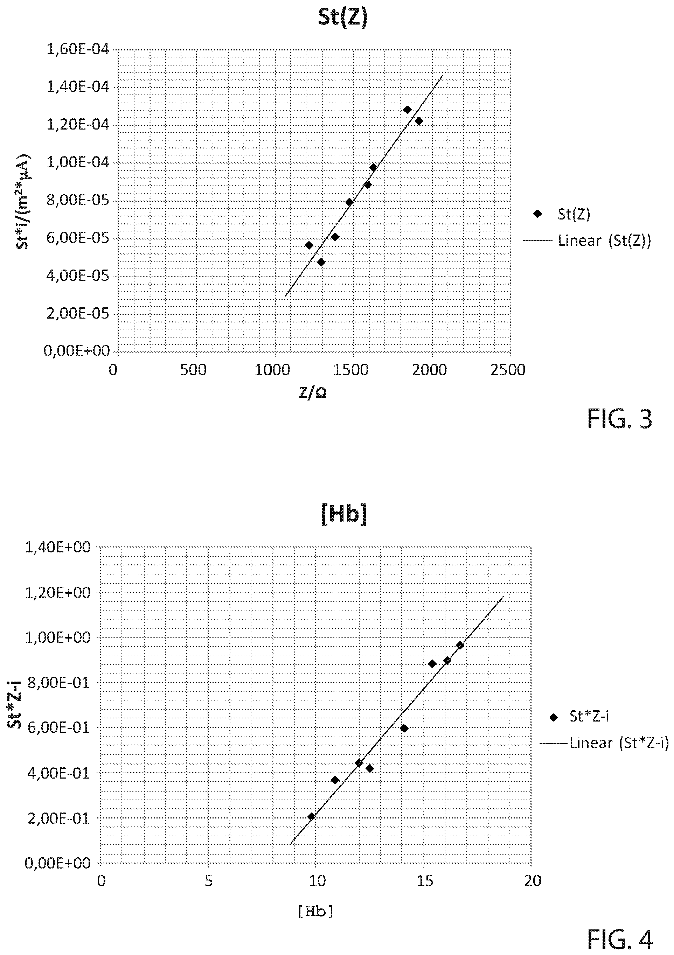

The surface factor St is linked to the current (i) and the impedance (Z) by the following relationship

$$S_t = (\alpha_3 Z + \beta_3) / i$$

where $\alpha_3$ and $\beta_3$ are respectively the slope and the intercept of the curve shown in Figure 3, where the least squares curve interpolating the points and indicated as "Linear (St (Z) ) " has slope $\alpha_3 = 1.17 \cdot 10^{-7}$ and intercept $\beta_3 = -9.46 \cdot 10^{-5}$.

In order to be able to measure the count of red blood cells (RC), haematocrit (HCT), mean corpuscular volume (MCV) and haemoglobin [Hb] concentration of an unknown blood sample, we proceed as indicated below.

Once the calibration curve is obtained, as shown in Figure 3, starting from the impedance and current values measured on the unknown sample of blood, the St value can be obtained through the equation indicated above.

Thereafter, the values of [Hb] (g/dL), HCT (%v/v) and RC (No. cells in $10^{12}$/L) can be obtained using the respective calibration curves as shown in Figure 4, 5 and 6.

The least squares curve interpolating the points and indicated as "Linear (St* Z-i)" has a slope $\alpha_0 = 0.111$ and intercept $\beta_0 = -0.893$ (Figure 4).

Therefore, the unknown measurement of the concentration of haemoglobin can be obtained by solving the following equation

$$[Hb] = (S_t \cdot Z - \beta_0 - i) / \alpha_0$$

where $\alpha_0$ and $\beta_0$ are respectively the slope and the intercept of the calibration curve shown in Figure 4.

Likewise, the HCT will be obtained from a relationship similar to the one above

$$HCT = (S_t \cdot Z - \beta_1 - i) / \alpha_1$$

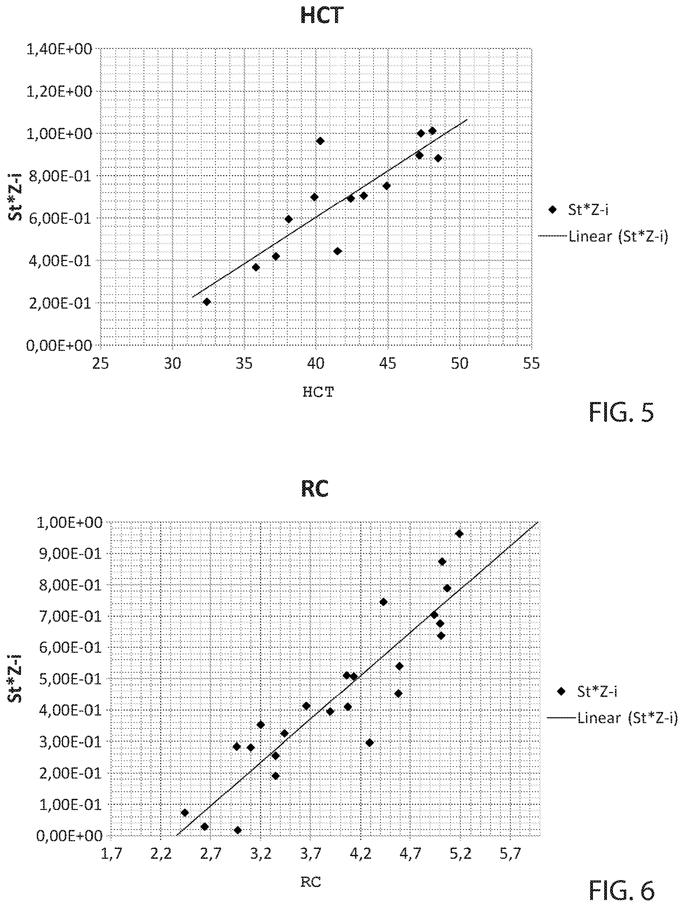

where $\alpha_1$ and $\beta_1$ are respectively the slope and the intercept of the calibration curve shown in Figure 5.

The least squares curve interpolating the points and indicated as "Linear (St* Z-i)" has slope $\alpha_0 = 0.045$ and intercept $\beta_0 = -1.226$ (Figure 5).

Similar to the relationships shown above, the count of red blood cells in the unknown sample can be obtained starting from the relationship

$$RC = (S_t \cdot Z - \beta_2 - i) / \alpha_2$$

where $\alpha_2$ and $\beta_2$ are respectively the slope and the intercept of the calibration curve shown in Figure 6. The least squares curve interpolating the points and indicated as "Linear (St* Z-i)" has slope $\alpha_2 = 0.224$ and intercept $\beta_2 = -0.433$ (Figure 6).

Finally, to obtain the MCV of the unknown sample, it will be sufficient to insert the measurements of the parameters obtained so far (St, HCT and RC) in the "inverse formula" of the formula of the total electroactive surface indicated above, obtaining in this case

$$MCV = 1 / (6 \cdot \sqrt{\pi}) \cdot (S_t / (\cdot RC \cdot HCT \cdot V))^{(3/2)}$$

Based on these observations, a second mathematical formula was also obtained to specifically measure haemoglobin, which directly correlates with both the current and impedance values measured by means of the same sensor.

$$[Hb] = Z (\alpha_4 \cdot i + \beta_4)$$

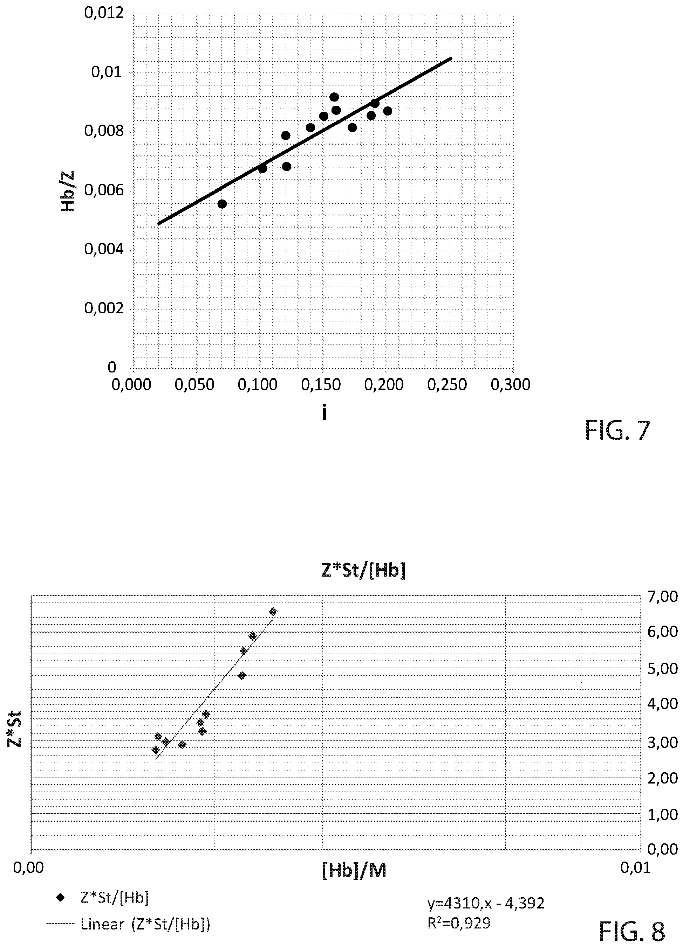

Where $\alpha_4$ and $\beta_4$ are respectively the slope and the intercept of the least squares interpolation curve shown in Figure 7.

In order to exemplify what has been set forth so far, the values obtained by measuring current and impedance of 8 unknown samples are shown in the following table.

| i | Z | St*i | St*Z-i | St_calc | Hb_calc | HCT_calc | RC_calc | MCV_calc |
|---|---|------|--------|---------|---------|----------|---------|----------|
| 0.160 | 1382 | 6.11E-05 | 0.36807 | 0.000416 | 11.8 | 35.7 | 3.9 | 70.5 |
| 0.213 | 1841 | 0.000128 | 0.89659 | 0.000564 | 15.5 | 45.0 | 5.3 | 48.1 |
| 0.179 | 1216 | 5.66E-05 | 0.20523 | 0.000264 | 9.3 | 29.5 | 2.9 | 74.0 |

(continued)

| i | Z | St*i | St*Z-i | St_calc | Hb_calc | HCT_calc | RC_calc | MCV_calc |
|---|---|------|--------|---------|---------|----------|---------|----------|
| 0.115 | 1293 | 4.75E-05 | 0.41960 | 0.000489 | 12.7 | 38.0 | 4.2 | 71.2 |
| 0.186 | 1472 | 7.95E-05 | 0.44354 | 0.000414 | 11.9 | 35.9 | 3.9 | 68.6 |
| 0.200 | 1626 | 9.77E-05 | 0.59471 | 0.000475 | 13.2 | 39.3 | 4.4 | 60.6 |
| 0.201 | 1915 | 0.000122 | 0.96418 | 0.000641 | 17.3 | 49.6 | 6.1 | 83.3 |
| 0.138 | 1589 | 8.86E-05 | 0.88262 | 0.000658 | 16.2 | 46.9 | 5.6 | 89.5 |

The type of matrix analysed in this context is capillary blood and venous blood. The signal (i and Z) obtained in the samples of capillary blood was different from that of the samples of venous blood in absolute values. The correlation factor between the two calibration curves (capillary and venous blood) was equal to 1.33.

In particular, the optimal sampling conditions were:

i. The test time of the electrochemical sensor must be less than 4 hours from the time of collection (for the collection of venous blood by butterfly and vacutainer system);
ii. The collection of capillary blood can be performed with or within the anticoagulant system (EDTA-K).

The measurements shown in the table above were obtained on samples of capillary blood. The venous blood had current and impedance values different from those of the capillary blood: the value of the ratio between the current obtained on the venous blood samples and the current of the capillary blood samples was equal to 1.33.

The quantification of the concentration of haemoglobin and of the other hemochromocytometric parameters was performed through the calibration curve between the samples processed with the reference method (using venous blood) and the samples processed with the electrochemical method (using capillary blood of the same patient).

The results obtained with this analytical approach are shown in the following table

| Sample No. | [Hb] [g/dL] Reference method | [Hb] [g/dL] Electrochemical method | Δ % |
|---|---|---|---|
| 1 | 10.9 | 11.1 | -1.61 |
| 2 | 16.1 | 16.4 | -2.02 |
| 3 | 16.1 | 17.6 | -9.30 |
| 4 | 9.8 | 9.4 | 3.80 |
| 5 | 12.5 | 11.6 | 7.22 |
| 6 | 12.0 | 11.8 | 1.34 |
| 7 | 14.1 | 13.4 | 5.18 |
| 8 | 15.3 | 14.4 | 6.18 |
| 9 | 13.2 | 14.4 | -9.09 |
| 10 | 16.7 | 17.1 | -4.63 |
| 11 | 12.6 | 14.5 | -15.02 |
| 12 | 15.4 | 16.3 | -5.74 |

The correlations obtained on 11 blood samples analysed first with the reference method for the hemochromocytometric examination were thus reported.

In Figure 8 it can be verified that the impedance measurement (Z) corrected by the electroactive surface factor (St), correlates with a fair significance with the concentration of haemoglobin expressed in mol/L (M). The scale of the abscissa axis is logarithmic. The haemoglobin values expressed in g/dL range from 10.4 to 16.2.

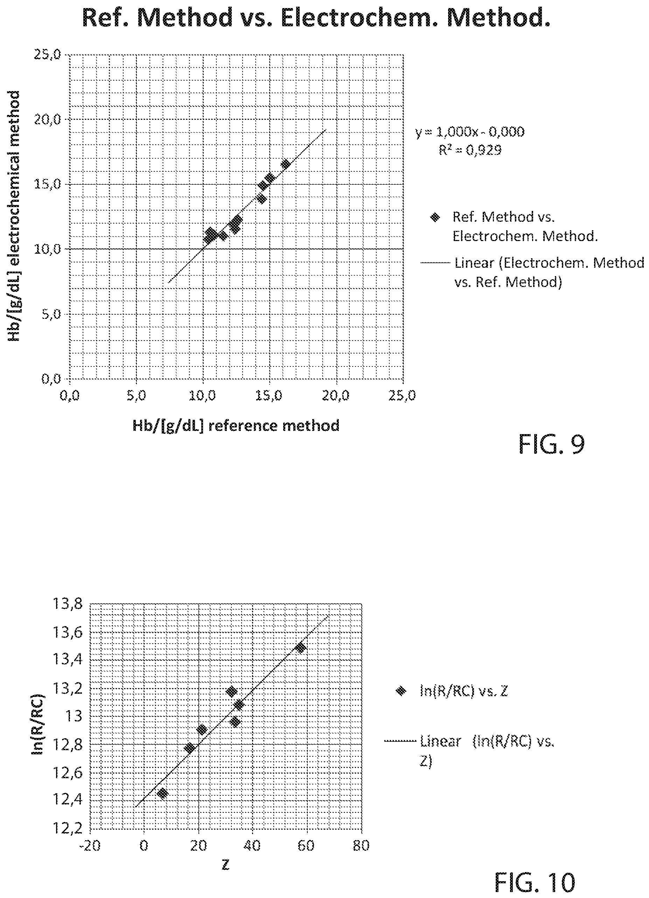

Figure 9 represents the comparison between the new measurement model of the concentration of blood haemoglobin and the concentration of haemoglobin determined with the reference method. Both concentrations are expressed in [g/dL]. The samples put in comparison are always the 11 of the model. There is a significant correlation. The maximum residual percentage is 6.9%.

Figure 10 shows as a natural logarithm the resistance measurements ($\Delta V/I$) normalized to the count of red blood cells contained in the volume of capillary blood deposited on the surface of the electrochemical cell (60 $\mu$l), as a function of the impedance measurements. The inverse proportionality between the count of red blood cells and the impedance can therefore be inferred therefrom. The samples taken into consideration are seven.

All this evidence leads to what currently represents the general model on which the analytical method is based. This model is an empirical relationship obtained starting from the correlable quantities such as Resistance, calculated in ohmic regime starting from the current measured in chronoamperometry (first part of the measurement) and the impedance obtained in the second part of the measurement.

In this sense, it should be specified that the sensor consists of two working electrodes on which the aforesaid measurements are carried out independently.

Advantages

Compared to the methods according to the prior art, the method according to the present invention has the following advantages:

- the device (sensor) is more miniaturised;
- the greater ease of industrialisation of the method and of the device;
- the reduced costs of the method and of the device;
- the volume of sample required is considerably smaller;
- no pre-treatment of the biological matrix is required;
- the time needed for the analysis is reduced;
- the execution of the method is simple and the device is easy to use;
- the measurements can be carried out both in the laboratory and at the point-of-care;
- the device is reagent-free;
- the device allows a precise and reliable measurement of the haemoglobin;
- the method can be applied to whole blood, capillary blood, serum and plasma, without the need for any pre-treatment of the biological matrix; the method can also be used in the presence of anticoagulants;
- this method can also allow the detection of all metalloproteins, in particular the iron (II) and iron (III) containing metalloproteins, e.g. myoglobin, or to dose the amount itself of iron within biological liquids;
- the same reading device can be used for the determination of other tests for which there are single-use diagnostic units (e.g. blood glucose).

Declaration pursuant to Art. 170bis(2) of the Italian Intellectual Property Code

The biological material of human origin used in the invention was acquired in accordance with the applicable legal provisions.

**Claims**

1. A method for detecting and/or quantifying, in a sample of blood or derivate biological liquid, a panel of haematological parameters comprising at least one parameter selected from the group consisting of the number of corpuscular elements in the blood or derivate biological liquid (RC), mean corpuscular volume of said corpuscular elements (MCV), percentage of the volume of sample occupied by the corpuscular elements (HCT) and amount of at least one substance comprising a protoporphyrin metal complex (Hb),

    comprising the steps of:

        - applying the sample of blood or derivate biological liquid to an electroanalytical sensor comprising at least one first working electrode, a reference electrode and a counterelectrode;
        - detecting by chronoamperometry a current signal which is a function of the amount of substance comprising the protoporphyrin metal complex in the blood or derivate biological liquid by means of the electroanalytical sensor, wherein an electrochemical mediator, which remains insoluble during the execution of the method, has been electrodeposited on said at least one first working electrode; and detecting by means of electrochemical impedance spectroscopy an impedance signal which is a function of at least the number of corpuscular elements in the blood or derivate biological liquid;
        - quantifying the at least one parameter of the panel by means of at least one specific mathematical formula wherein the at least one parameter is a function of the current signal (i) and the impedance signal (Z),

wherein in the specific mathematical formula the at least one parameter is a function of the total surface of the corpuscular elements (St), wherein St is a function of the impedance signal (Z) and the current signal (i) by means of equation

$$S_t \;=\; (\alpha_3 \cdot Z + \beta_3)/i$$

wherein $\alpha_3$ and $\beta_3$ are respectively the angular coefficient and the intercept of a calibration line obtained experimentally and traced on a graph wherein Z is on the abscissae axys and St • i is on the ordinates axis.

2. A method for detecting and/or quantifying, in a sample of blood or derivate biological liquid, an amount of at least one substance comprising a protoporphyrin metal complex (Hb),

   comprising the steps of:

   - applying the sample of blood or derivate biological liquid to an electroanalytical sensor comprising at least one first working electrode, a reference electrode and a counterelectrode;
   - detecting by chronoamperometry a current signal which is a function of the amount of substance comprising the protoporphyrin metal complex in the blood or derivate biological liquid by means of the electroanalytical sensor, wherein an electrochemical mediator, which remains insoluble during the execution of the method, has been electrodeposited on said at least one first working electrode; and detecting by means of electrochemical impedance spectroscopy an impedance signal which is a function of at least the number of corpuscular elements in the blood or derivate biological liquid;
   - quantifying the amount of at least one substance comprising a protoporphyrin metal complex (Hb) by means of at least one specific mathematical formula, wherein the amount of at least one substance comprising protoporphyrin metal complex (Hb) is a function of the current signal (i) and the impedance signal (Z), wherein the specific mathematical formula is:

   $$[Hb] \;=\; Z \cdot (\alpha_4 \cdot i + \beta_4)$$

   wherein [Hb] is the concentration of the substance comprising the protoporphyrin metal complex; and wherein $\alpha_4$ and $\beta_4$ are respectively the slope and the intercept of the fitting line to the least squares obtained placing the ratio between the concentration of the substance comprising the protoporphyrin metal complex and the impedance on the ordinate axis, and the current intensity on the abscissa axis.

3. The method according to claim 1, wherein

   $$S_t \;=\; RC \cdot HCT \cdot \sqrt[3]{(36\pi \ (MCV)^2)}$$

   wherein RC is the number of said corpuscular elements [n. • $10^{12}$ /l]
   HCT is the relative corpuscular volume of said corpuscular elements [%V/V]
   V is the volume of the sample of blood or derivate biological liquid applied to the sensor
   MCV is the mean corpuscular volume [fl]
   and St is a function of the impedance signal and the current signal by means of the equation

   $$S_t \;=\; (\alpha_3 \cdot Z + \beta_3)/i$$

   wherein $\alpha_3$ and $\beta_3$ are respectively the slope and the intercept of a calibration curve obtained experimentally and traced on a graph wherein Z ($\Omega$) is on the abscissa axis and St • i ($m^2$ • $\mu A$) is on the ordinate axis.

4. The method according to claim 1 or 3, wherein the parameter amount of at least one substance comprising a protoporphyrin metal complex (Hb) is measured, wherein the specific mathematical formula is:

   $$[Hb] \;=\; (S_t \cdot Z - \beta_0 - i)/\alpha_0$$

wherein [Hb] is the concentration of the substance comprising the protoporphyrin metal complex; and $\alpha_0$ and $\beta_0$ are respectively the slope and the intercept of a calibration curve obtained experimentally and traced on a graph wherein [Hb] (g/dL) is on the abscissa axis and (St · Z-i) (m$^2$ * Ohm - $\mu$A) is on the ordinate axis.

5. The method according to any of claims 1, 3 or 4, wherein the parameter percentage of volume occupied by corpuscular elements/volume of the sample is measured, wherein the specific mathematical formula is:

$$\mathrm{HCT} = (S_t \cdot Z - \beta_1 - i)/\alpha_1$$

wherein HCT is the relative corpuscular volume of said corpuscular elements; and $\alpha_1$ and $\beta_1$ are respectively the slope and the intercept of a calibration curve obtained experimentally and traced on a graph wherein HCT (%v/v) is on the abscissa axis and (St • Z-i) (m$^2$ * Ohm - $\mu$A) is on the ordinate axis.

6. The method according to any of claims from 1 or 3 to 5, wherein the parameter number of corpuscular elements in the blood or derivate biological liquid is measured, wherein the specific mathematical formula is:

$$\mathrm{RC} = (S_t \cdot Z - \beta_2 - i)/\alpha_2$$

wherein RC is the number of said corpuscular elements; and $\alpha_2$ and $\beta_2$ are respectively the slope and the intercept of a calibration curve obtained experimentally and traced on a graph wherein RC (n. corpuscular elements · 10$^{12}$/l) is on the abscissa axis and ($S_t$ · Z-i) (m$^2$ * Ohm - $\mu$A) is on the ordinate axis.

7. The method according to any of claims from 1 or 3 to 6, wherein the parameter mean corpuscular volume in the blood or derivate biological liquid is measured, wherein the specific mathematical formula is:

$$\mathrm{MCV} = 1/(6\ \sqrt{\pi}) \bullet (S_t/(RC \cdot HCT \cdot V))^{\wedge}(3/2)$$

wherein MCV is the mean corpuscular volume.

8. The method according to any of claims from 1 or 3 to 7, wherein all the parameters of the panel are measured.

9. The method according to any of the preceding claims, wherein the corpuscular elements, are cell elements selected from the group consisting of erythrocytes, reticulocytes ed erythroblasts.

10. The method according to any of the preceding claims, wherein the protoporphyrin metal complex is an iron protoporphyrin complex, preferably haemoglobin or myoglobin, or a zinc protoporphyrin complex, preferably zinc porphyrin.

11. The method according to any of claims 1 or 3 to 10, wherein HCT is haematocrit, RC is the number of erythrocytes, Hb is haemoglobin.

12. The method according to any of the preceding claims, wherein the electrochemical mediator is selected from the group consisting of methylene blue, methyl viologen, o-cresol indophenol, 2-6 dibromophenol-indophenol, toluidine blue, haematoxylin/eosin, periodic acid with Schiff stain.

13. The method according to claim 12, wherein the electrochemical mediator is methylene blue.

14. The method according to any of the preceding claims, wherein the sample of blood or derivate biological liquid has a volume smaller or equivalent to 50 $\mu$l, preferably smaller than 15 $\mu$l.

15. The method according to any of the preceding claims, wherein the step of detecting by chronoamperometry is carried out at a potential from -1V to +1V, preferably at a potential of about 0.55 V.

**Patentansprüche**

1. Verfahren zum Detektieren und/oder zur Quantifizieren einer Gruppe von hämatologischen Parametern in einer Probe von Blut oder einer abgeleiteten biologischen Flüssigkeit, umfassend mindestens einen Parameter, ausgewählt aus der Gruppe bestehend aus der Anzahl der korpuskulären Elemente in dem Blut oder der abgeleiteten biologischen Flüssigkeit (RC), dem mittleren korpuskulären Volumen der korpuskulären Elemente (MCV), dem Prozentsatz des von den korpuskulären Elementen (HCT) eingenommenen Probenvolumens und der Menge mindestens einer Substanz, die einen Protoporphyrin-Metallkomplex (Hb) umfasst,

   umfassend folgende Schritte:

   - Aufbringen der Probe von Blut oder einer abgeleiteten biologischen Flüssigkeit auf einen elektroanalytischen Sensor, umfassend mindestens eine erste Arbeitselektrode, eine Referenzelektrode und eine Gegenelektrode;
   - Detektieren eines Stromsignals durch Chronoamperometrie, das eine Funktion der Menge der Substanz ist, die den Protoporphyrin-Metallkomplex in dem Blut oder der abgeleiteten biologischen Flüssigkeit umfasst, mittels des elektroanalytischen Sensors, wobei ein elektrochemischer Mediator, der während der Durchführung des Verfahrens unlöslich bleibt, auf der mindestens einen ersten Arbeitselektrode elektrochemisch abgeschieden wurde; und Detektieren eines Impedanzsignals mittels elektrochemischer Impedanzspektroskopie, das eine Funktion von mindestens der Anzahl von korpuskulären Elementen in dem Blut oder der abgeleiteten biologischen Flüssigkeit ist;
   - Quantifizieren des mindestens einen Parameters des Panels mittels mindestens einer spezifischen mathematischen Formel, wobei der mindestens eine Parameter eine Funktion des Stromsignals (i) und des Impedanzsignals (Z) ist,

   wobei in der spezifischen mathematischen Formel der mindestens eine Parameter eine Funktion der Gesamtoberfläche der korpuskulären Elemente (St) ist, wobei St eine Funktion des Impedanzsignals (Z) und des Stromsignals (i) mittels der Gleichung

   $$S_t = (\alpha_3 \cdot Z + \beta_3) / i$$

   ist

   wobei $\alpha_3$ und $\beta_3$ der Winkelkoeffizient bzw. der Achsenabschnitt einer experimentell erhaltenen Kalibrierungslinie sind, die auf einem Diagramm aufgetragen ist, in dem Z auf der Abszissenachse und St · i auf der Ordinatenachse liegt.

2. Verfahren zum Detektieren und/oder zur Quantifizieren einer Menge mindestens einer Substanz, die einen Protoporphyrin-Metallkomplex (Hb) umfasst, in einer Probe von Blut oder einer abgeleiteten biologischen Flüssigkeit,

   umfassend folgende Schritte:

   - Aufbringen der Probe von Blut oder einer abgeleiteten biologischen Flüssigkeit auf einen elektroanalytischen Sensor, umfassend mindestens eine erste Arbeitselektrode, eine Referenzelektrode und eine Gegenelektrode;
   - Detektieren eines Stromsignals durch Chronoamperometrie, das eine Funktion der Menge der Substanz ist, die den Protoporphyrin-Metallkomplex in dem Blut oder der abgeleiteten biologischen Flüssigkeit umfasst, mittels des elektroanalytischen Sensors, wobei ein elektrochemischer Mediator, der während der Durchführung des Verfahrens unlöslich bleibt, auf der mindestens einen ersten Arbeitselektrode elektrochemisch abgeschieden wurde; und Detektieren eines Impedanzsignals mittels elektrochemischer Impedanzspektroskopie, das eine Funktion von mindestens der Anzahl von korpuskulären Elementen in dem Blut oder der abgeleiteten biologischen Flüssigkeit ist;
   - Quantifizieren der Menge mindestens einer Substanz, die einen Protoporphyrin-Metallkomplex (Hb) umfasst, mittels mindestens einer spezifischen mathematischen Formel, wobei die Menge mindestens einer Substanz, die einen Protoporphyrin-Metallkomplex (Hb) umfasst, eine Funktion des Stromsignals (i) und des Impedanzsignals (Z) ist, wobei die spezifische mathematische Formel lautet:

   $$[Hb] = Z \cdot (\alpha_4 \cdot i + \beta_4)$$

wobei [Hb] die Konzentration der Substanz ist, die den Protoporphyrin-Metallkomplex umfasst; und

wobei $\alpha_4$ und $\beta_4$ die Steigung bzw. der Achsenabschnitt der Anpassungslinie an die kleinsten Quadrate sind, die man erhält, indem man das Verhältnis zwischen der Konzentration der Substanz, die den Protoporphyrin-Metallkomplex umfasst, und der Impedanz auf der Ordinatenachse und der Stromstärke auf der Abszissenachse einsetzt.

3. Verfahren nach Anspruch 1, wobei

$$S_t = RC \cdot HCT \cdot \sqrt[3]{(36\pi\ (MCV)^2)}$$

wobei RC die Anzahl der korpuskulären Elemente [n. $\cdot 10^{12}$ /l] ist

HCT das relative korpuskuläre Volumen der korpuskulären Elemente [%V/V] ist

V das Volumen der auf den Sensor aufgebrachten Probe von Blut oder einer abgeleiteten biologischen Flüssigkeit ist

MCV das mittlere korpuskuläre Volumen [f1] ist

und St eine Funktion des Impedanzsignals und des Stromsignals mittels der Gleichung

$$S_t = (\alpha_3 \cdot Z + \beta_3)\ /i$$

ist

wobei $\alpha_3$ und $\beta_3$ die Steigung bzw. der Achsenabschnitt einer experimentell erhaltenen Kalibrierungskurve sind, die in einem Diagramm eingetragen ist, in dem Z ($\Omega$) auf der Abszissenachse und St $\cdot$ i (m$^2$ $\cdot$ $\mu$A) auf der Ordinatenachse liegt.

4. Verfahren nach Anspruch 1 oder 3, wobei der Parameter Menge mindestens einer Substanz, die einen Protoporphyrin-Metallkomplex (Hb) umfasst, gemessen wird, wobei die spezifische mathematische Formel lautet:

$$[Hb] = (S_t \cdot Z - \beta_0 - i)\ /\alpha_0$$

wobei [Hb] die Konzentration der Substanz ist, die den Protoporphyrin-Metallkomplex umfasst; und

$\alpha_0$ und $\beta_0$ die Steigung bzw. der Achsenabschnitt einer experimentell erhaltenen Kalibrierungskurve sind, die in einem Diagramm eingetragen ist, in dem [Hb] (g/dL) auf der Abszissenachse und (St $\cdot$ Z-i) (m$^2$ * Ohm - $\mu$A) auf der Ordinatenachse liegt.

5. Verfahren nach einem der Ansprüche 1, 3 oder 4, wobei der Parameter Prozentsatz des von korpuskulären Elementen belegten Volumens/Volumen der Probe gemessen wird, wobei die spezifische mathematische Formel lautet:

$$[Hb] = (S_t \cdot Z - \beta_1 - i)\ /\alpha_1$$

wobei HCT das relative korpuskuläre Volumen der korpuskulären Elemente ist; und

$\alpha_1$ und $\beta_1$ die Steigung bzw. der Achsenabschnitt einer experimentell ermittelten Kalibrierungskurve, die in ein Diagramm eingetragen wird, in dem HCT (%v/v) auf der Abszissenachse und (St $\cdot$ Z-i) (m$^2$ * Ohm - $\mu$A) auf der Ordinatenachse liegt.

6. Verfahren nach einem der Ansprüche 1 oder 3 bis 5, wobei der Parameter Anzahl der korpuskulären Elemente im Blut oder in der abgeleiteten biologischen Flüssigkeit gemessen wird, wobei die spezifische mathematische Formel lautet:

$$RC = (S_t \cdot Z - \beta_2 - i)\ /\alpha_2$$

wobei RC die Anzahl der genannten korpuskulären Elemente ist; und

$\alpha_2$ und $\beta_2$ die Steigung bzw. der Achsenabschnitt einer experimentell ermittelten Kalibrierungskurve sind, die in ein Diagramm eingetragen wird, in dem RC (n. korpuskuläre Elemente $\cdot 10^{12}$/l) auf der Abszissenachse und (St $\cdot$ Z-i) (m$^2$ * Ohm - $\mu$A) auf der Ordinatenachse liegt.

**7.** Verfahren nach einem der Ansprüche 1 oder 3 bis 6, wobei der Parameter mittleres korpuskuläres Volumen in dem Blut oder der abgeleiteten biologischen Flüssigkeit gemessen wird, wobei die spezifische mathematische Formel lautet:

$$\text{MCV} = 1/(6\ \sqrt{\pi})\ \bullet\ (S_t/(RC\bullet HCT\bullet V))^{(3/2)}$$

wobei MCV das mittlere korpuskuläre Volumen ist.

**8.** Verfahren nach einem der Ansprüche 1 oder 3 bis 7, wobei alle Parameter des Panels gemessen werden.

**9.** Verfahren nach einem der vorstehenden Ansprüche, wobei die korpuskulären Elemente Zellelemente sind, ausgewählt aus der Gruppe bestehend aus Erythrozyten, Retikulozyten und Erythroblasten.

**10.** Verfahren nach einem der vorstehenden Ansprüche, wobei der Protoporphyrin-Metallkomplex ein Eisen-Protoporphyrin-Komplex, vorzugsweise Hämoglobin oder Myoglobin, oder ein Zink-Protoporphyrin-Komplex, vorzugsweise Zinkporphyrin, ist.

**11.** Verfahren nach einem der Ansprüche 1 oder 3 bis 10, wobei HCT der Hämatokrit, RC die Anzahl der Erythrozyten und Hb das Hämoglobin ist.

**12.** Verfahren nach einem der vorstehenden Ansprüche, wobei der elektrochemische Mediator ausgewählt aus der Gruppe bestehend aus Methylenblau, Methylviologen, o-Kresol-Indophenol, 2-6-Dibromphenol-Indophenol, Toluidinblau, Hämatoxylin/Eosin, periodische Säure mit Schiffscher Färbung.

**13.** Verfahren nach Anspruch 12, wobei der elektrochemische Mediator Methylenblau ist.

**14.** Verfahren nach einem der vorstehenden Ansprüche, wobei die Probe von Blut oder einer abgeleiteten biologischen Flüssigkeit ein Volumen von kleiner gleich 50 $\mu$l, vorzugsweise kleiner 15 $\mu$l, aufweist.

**15.** Verfahren nach einem der vorstehenden Ansprüche, wobei der Schritt des Detektierens durch Chronoamperometrie bei einem Potential von -1 V bis +1 V, vorzugsweise bei einem Potential von etwa 0,55 V, durchgeführt wird.

**Revendications**

**1.** Procédé de détection et/ou de quantification, dans un échantillon sanguin ou de liquide biologique dérivé, d'un panel de paramètres hématologiques comprenant au moins un paramètre choisi dans le groupe constitué du nombre d'éléments corpusculaires dans le sang ou le liquide biologique dérivé (RC), du volume corpusculaire moyen desdits éléments corpusculaires (MCV), du pourcentage du volume de l'échantillon occupé par les éléments corpusculaires (HCT) et de la quantité d'au moins une substance comprenant un complexe métallique de protoporphyrine (Hb),

comprenant les étapes consistant à :

- appliquer l'échantillon sanguin ou de liquide biologique dérivé à un capteur électroanalytique comprenant au moins une première électrode de travail, une électrode de référence et une contreélectrode ;
- détecter par chronoampérométrie un signal de courant qui est fonction de la quantité de substance comprenant le complexe métallique de protoporphyrine dans le sang ou le liquide biologique dérivé au moyen du capteur électroanalytique, un médiateur électrochimique, restant insoluble pendant l'exécution du procédé, a été électrodéposé sur ladite au moins une première électrode de travail ; et détecter par spectroscopie d'impédance électrochimique un signal d'impédance qui est fonction au moins du nombre d'éléments corpusculaires dans le sang ou le liquide biologique dérivé ;
- quantifier l'au moins un paramètre du panel au moyen d'au moins une formule mathématique spécifique, où l'au moins un paramètre est fonction du signal de courant (i) et du signal d'impédance (Z),

dans la formule mathématique spécifique, l'au moins un paramètre est fonction de la surface totale des éléments corpusculaires (St), St étant fonction du signal d'impédance (Z) et du signal de courant (i) au moyen de l'équation suivante

$$S_t = (\alpha_3 \cdot Z + \beta_3) / i$$

où $\alpha_3$ et $\beta_3$ sont respectivement le coefficient angulaire et l'ordonnée à l'origine d'une ligne d'étalonnage obtenue expérimentalement et tracée sur un graphique où Z est sur l'axe des abscisses et St • i sur l'axe des ordonnées.

2. Procédé de détection et/ou de quantification, dans un échantillon sanguin ou de liquide biologique dérivé, d'une quantité d'au moins une substance comprenant un complexe métallique de protoporphyrine (Hb),

comprenant les étapes consistant à :

- appliquer l'échantillon sanguin ou de liquide biologique dérivé à un capteur électroanalytique comprenant au moins une première électrode de travail, une électrode de référence et une contreélectrode ;
- détecter par chronoampérométrie un signal de courant qui est fonction de la quantité de substance comprenant le complexe métallique de protoporphyrine dans le sang ou le liquide biologique dérivé au moyen du capteur électroanalytique, un médiateur électrochimique, restant insoluble pendant l'exécution du procédé, a été électrodéposé sur ladite au moins une première électrode de travail ; et détecter par spectroscopie d'impédance électrochimique un signal d'impédance qui est fonction au moins du nombre d'éléments corpusculaires dans le sang ou le liquide biologique dérivé ;
- quantifier la quantité d'au moins une substance comprenant un complexe métallique de protoporphyrine (Hb) au moyen d'au moins une formule mathématique spécifique, la quantité d'au moins une substance comprenant un complexe métallique de protoporphyrine (Hb) étant fonction du signal de courant (i) et du signal d'impédance (Z), la formule mathématique spécifique étant :

$$[Hb] = Z \cdot (\alpha_4 \cdot i + \beta_4)$$

où [Hb] est la concentration de la substance comprenant le complexe métallique de protoporphyrine ; et où $\alpha_4$ et $\beta_4$ sont respectivement la pente et l'ordonnée à l'origine de la droite d'ajustement aux moindres carrés obtenue en plaçant le rapport entre la concentration de la substance comprenant le complexe métallique de protoporphyrine et l'impédance sur l'axe des ordonnées, et l'intensité du courant sur l'axe des abscisses.

3. Procédé selon la revendication 1, dans lequel

$$S_t = RC \cdot HCT \cdot \sqrt[3]{(36\pi (MCV)^2)}$$

où RC est le nombre desdits éléments corpusculaires [nb • $10^{12}$ /l]
HCT est le volume corpusculaire relatif desdits éléments corpusculaires [% V/V]
V est le volume de l'échantillon sanguin ou de liquide biologique dérivé appliqué au capteur.
MCV est le volume corpusculaire moyen [f1].
et St est fonction du signal d'impédance et du signal de courant au moyen de l'équation suivante

$$S_t = (\alpha_3 \cdot Z + \beta_3) / i$$

où $\alpha_3$ et $\beta_3$ sont respectivement la pente et l'ordonnée à l'origine d'une courbe d'étalonnage obtenue expérimentalement et tracée sur un graphique où Z ($\Omega$) est sur l'axe des abscisses et St • i ($m^2$ • $\mu$A) sur l'axe des ordonnées.

4. Procédé selon la revendication 1 ou 3, la quantité paramétrique d'au moins une substance comprenant un complexe métallique de protoporphyrine (Hb) étant mesurée, la formule mathématique spécifique étant :

$$[Hb] = (S_t \cdot Z - \beta_0 - i) / \alpha_0$$

où [Hb] est la concentration de la substance comprenant le complexe métallique de protoporphyrine ; et $\alpha_0$ et $\beta_0$ sont respectivement la pente et l'ordonnée à l'origine d'une courbe d'étalonnage obtenue expérimentalement et tracée sur un graphique où [Hb] (g/dL) est sur l'axe des abscisses et (St • Z-i) ($m^2$ * Ohm - $\mu$A) est sur l'axe des ordonnées.

5. Procédé selon l'une quelconque des revendications 1, 3 ou 4, le paramètre pourcentage du volume occupé par les éléments corpusculaires/volume de l'échantillon étant mesuré, la formule mathématique spécifique étant la suivante :

$$[\text{Hb}] = (S_t \bullet Z - \beta_1 - i) / \alpha_1$$

où HCT est le volume corpusculaire relatif desdits éléments corpusculaires ; et
$\alpha_1$ et $\beta_1$ sont respectivement la pente et l'ordonnée à l'origine d'une courbe d'étalonnage obtenue expérimentalement et tracée sur un graphique où HCT (% v/v) est sur l'axe des abscisses et $(St \bullet Z-i)$ ($m^2$ * Ohm - $\mu$A) est sur l'axe des ordonnées.

6. Procédé selon l'une quelconque des revendications 1 ou 3 à 5, le paramètre nombre d'éléments corpusculaires dans le sang ou le liquide biologique dérivé étant mesuré, la formule mathématique spécifique étant la suivante :

$$RC = (S_t \bullet Z - \beta_2 - i) / \alpha_2$$

où RC est le nombre desdits éléments corpusculaires ; et
$\alpha_2$ et $\beta_2$ sont respectivement la pente et l'ordonnée à l'origine d'une courbe d'étalonnage obtenue expérimentalement et tracée sur un graphique où RC (nb éléments corpusculaires • $10^{12}$/l) est sur l'axe des abscisses et $(St \bullet Z-i)$ ($m^2$ * Ohm - $\mu$A) est sur l'axe des ordonnées.

7. Procédé selon l'une quelconque des revendications 1 ou 3 à 6, le paramètre volume corpusculaire moyen dans le sang ou le liquide biologique dérivé étant mesuré, la formule mathématique spécifique étant la suivante :

$$MCV = 1/(6 \; \sqrt{\pi}) \bullet (S_t/(RC \bullet HCT \bullet V))^{(3/2)}$$

où MCV est le volume corpusculaire moyen.

8. Procédé selon l'une quelconque des revendications 1 ou 3 à 7, tous les paramètres du panel étant mesurés.

9. Procédé selon l'une quelconque des revendications précédentes, les éléments corpusculaires étant des éléments cellulaires choisis dans le groupe constitué par les érythrocytes, les réticulocytes et les érythroblastes.

10. Procédé selon l'une quelconque des revendications précédentes, le complexe métallique de protoporphyrine étant un complexe de protoporphyrine de fer, de préférence l'hémoglobine ou la myoglobine, ou un complexe de protoporphyrine de zinc, de préférence la porphyrine de zinc.

11. Procédé selon l'une quelconque des revendications 1 ou 3 à 10, HCT étant l'hématocrite, RC étant le nombre d'érythrocytes, Hb étant l'hémoglobine.

12. Procédé selon l'une quelconque des revendications précédentes, le médiateur électrochimique étant choisi dans le groupe constitué par le bleu de méthylène, le méthyl-viologène, l'o-crésol-indophénol, le 2-6 dibromophénol-indophénol, le bleu de toluidine, l'hématoxyline/éosine, l'acide périodique avec la coloration de Schiff.

13. Procédé selon la revendication 12, le médiateur électrochimique étant le bleu de méthylène.

14. Procédé selon l'une quelconque des revendications précédentes, l'échantillon sanguin ou de liquide biologique dérivé ayant un volume inférieur ou équivalent à 50 $\mu$l, de préférence inférieur à 15 $\mu$l.

15. Procédé selon l'une quelconque des revendications précédentes, l'étape de détection par chronoampérométrie étant effectuée à un potentiel de - 1 V à + 1 V, de préférence à un potentiel d'environ 0,55 V.

FIG. 1

| $E_{begin}$ = -0.6 V | $E_{vertex1}$ = 1 V | $E_{vertex2}$ = -0.6 V | $E_{step}$ = 0.005 V |
|---|---|---|---|
| Scan rate = 0.1 V/s | | Number of scans = 20 | |

FIG. 2

FIG. 3

FIG. 4

# HCT

**FIG. 5**

# RC

**FIG. 6**

FIG. 7

Z*St/[Hb]

◆ Z*St/[Hb]

—— Linear (Z*St/[Hb])

y=4310,x - 4,392
R²=0,929

FIG. 8

## Ref. Method vs. Electrochem. Method.

$y = 1,000x - 0,000$
$R^2 = 0,929$

◆ Ref. Method vs.
Electrochem. Method.

—— Linear (Electrochem. Method
vs. Ref. Method)

**FIG. 9**

◆ ln(R/RC) vs. Z

—— Linear (ln(R/RC) vs.
Z)

**FIG. 10**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 102023000001014
- US 2013062221 A1